# EUROPEAN PATENT SPECIFICATION

(11) **EP 3 965 773 B1**
(45) Date of publication and mention of the grant of the patent: **31.12.2025**
(21) Application number: 20802732.6
(22) Date of filing: 08.05.2020
(51) Int. Cl.: C07C 401/00, C07J 9/00, C07J 53/00, A61K 31/593

(54) **NOVEL METHOD FOR SYNTHESIZING 25-OH CHOLESTEROL/CALCIFEDIOL FROM PHYTOSTEROL**
NEUARTIGES VERFAHREN ZUR SYNTHESE VON 25-OH CHOLESTERIN/CALCIFEDIOL AUS PHYTOSTEROL
NOUVEAU PROCÉDÉ DE SYNTHÈSE DE 25-OH CHOLESTÉROL/CALCIFÉDIOL À PARTIR DE PHYTOSTÉROL

(30) Priority: 09.05.2019 IN 201921018561
(43) Date of publication of application: 16.03.2022
(73) Proprietor: Fermenta Biotech Limited, Thane (West) Maharashtra 400610 (IN)
(72) Inventor: DATLA, Anupama, Mumbai, Maharashtra 400049 (IN); NAGRE, Prashant, Thane (West), Maharashtra 400607 (IN); TAMORE, Jagdish, Thane, Maharashtra 400303 (IN); PRABHU, Manojkumar Sadanand, Thane Dist, Maharashtra 421301 (IN); KADAM, Sachin Vasant, Thane (West), Maharashtra 400601 (IN); SHIRSATH, Amol, Maharashtra (IN)
(74) Representative: Isarpatent
(86) International application number: PCT/IN2020/050416
(87) International publication number: WO 2020/225830

(56) References cited:
- CN-A- 109 021 059
- US-A- 4 069 321
- KUREK-TYRLIK ET AL.: "Synthesis of 17- epi -Calcitriol from a Common Androstane Derivative, Involving the Ring B Photochemical Opening and the Intermediate Triene Ozonolysis", J. ORG. CHEM, vol. 70, 2005, pages 8513 - 8521, XP055759758, DOI: https://doi.org/10.1021/jo051357u.
- SUSAN D. VAN ARNUM ET AL.: "Process Control Limits from a Laboratory Study on the Ni(0)-Mediated Coupling of Ethyl Acrylate with a C-22 Steroidal Iodide: A Case Study on the Role of Experimental Design in Highly Developed Processes", ORGANIC PROCESS RESEARCH & DEVELOPMENT, vol. 8, 2004, pages 769 - 776, XP055759767, DOI: https://doi.org/10.1021/op049908q.
- MARTIN KARPF ET AL.: "Synthesis of Ficisterol- The first natural sterol with 23-ethyl substitution", TETRAHEDRON LETTERS, 1980, XP055759775
- HAJIME NAGANO ET AL.: "Stereoselective synthesis of 24-alkyl-22-hydroxysterols based on chelation controlled radical reactions", J. CHEM. SOC., PERKIN TRANS., vol. 1, 2001, pages 174 - 182, XP055759779, DOI: 10.1039/b0007139k

## Description

### Technical filed:

The present invention relates to novel method for synthesizing vegan 25-OH cholesterol/Calcifediol from inexpensive crude phytosterol.

### Background and prior art:

The metabolite of vitamin D3, 25-hydroxycholecalciferol(Calcifediol) is more potent anti- rachitic agent than vitamin D itself therefore development of a facile method for the synthesis of vitamin D3 and its analogue Calcifediol is highly important. Further, 25-hydroxy cholesterol is an important raw material for the synthesis of 25-hydroxy vitamin D3, also known as calcifediol, is the active metabolite of vitamin D3, has a stronger physiological activity, and does not need to go through the liver metabolism.

The 25-hydroxy vitamin D3 has the following unique functions. Vitamin D3 in some human or animals cannot be directly converted into 25-hydroxy vitamin D3 due to liver function disorder. Since the 25-hydroxy vitamin D3, as an active substance, bypasses the liver transformation and hence can be directly supplied for human or animals for absorption. The 25-hydroxy vitamin D3 in animals can promote the bone development of poultry, maximize the bone mineral density, reduce chick mortality, reduce osteoporosis and cage layer fatigue, improve quality of egg shells, reduce the breakage rate of egg shells, increase the hatching rate and prolong the egg production cycle. When compared with vitamin D3, absorption of the 25-hydroxy vitamin D3 is less affected by intestinal damage; the content of the 25-hydroxy vitamin D3 in plasma is a direct indicator for the nutritional status of vitamin D3.

Although, the market requirement of 25-hydroxy cholesterol is more, however, the availability of the same is less due to the difficulty in the existing production processes.

The preparation of 25-hydroxycholecalciferol had been reported from 25-hydroxy cholesterol which in turn is prepared from pure stigmasterol which is again isolated from crude phytosterol. Phytosterol is a mixture of sterols i.e. a mixture of stigmasterol, beta-sitosterol, campesterol and stigmastenol.

Stigmasterol is an unsaturated sterol, having one double bond in the sterol ring structure and one double bond in the side chain. Beta sitosterol is an unsaturated sterol with one double bond in sterol ring structure. Campesterol is structurally similar to beta sitosterol but has methyl group substituent at C24 position instead of an ethyl group. Stigmastenol is a saturated sterol both in ring structure and in the side chain.

There is very limited literature available on methods of preparation of 25-hydroxycholesterol and its esters starting from the naturally occurring phytosterol. US3822254 discloses a process for the preparation of 25-hydroxycholesterol and its esters starting from the naturally occurring (readily available and inexpensive) starting material, stigmasterol which is isolated commercially from Soybeans. The synthesis encompasses, as key steps, the protection of the 3-hydroxy-delta 5-function by formation of an i-steroid, cleavage of the 22, 23-double bond, and introduction of the properly substituted 5-carbon fragment to afford the 25-hydroxycholesterol side chain. The synthesis of 25-hydroxycholesterol from pure stigmasterol as reported in US3822254 is shown in scheme 1.

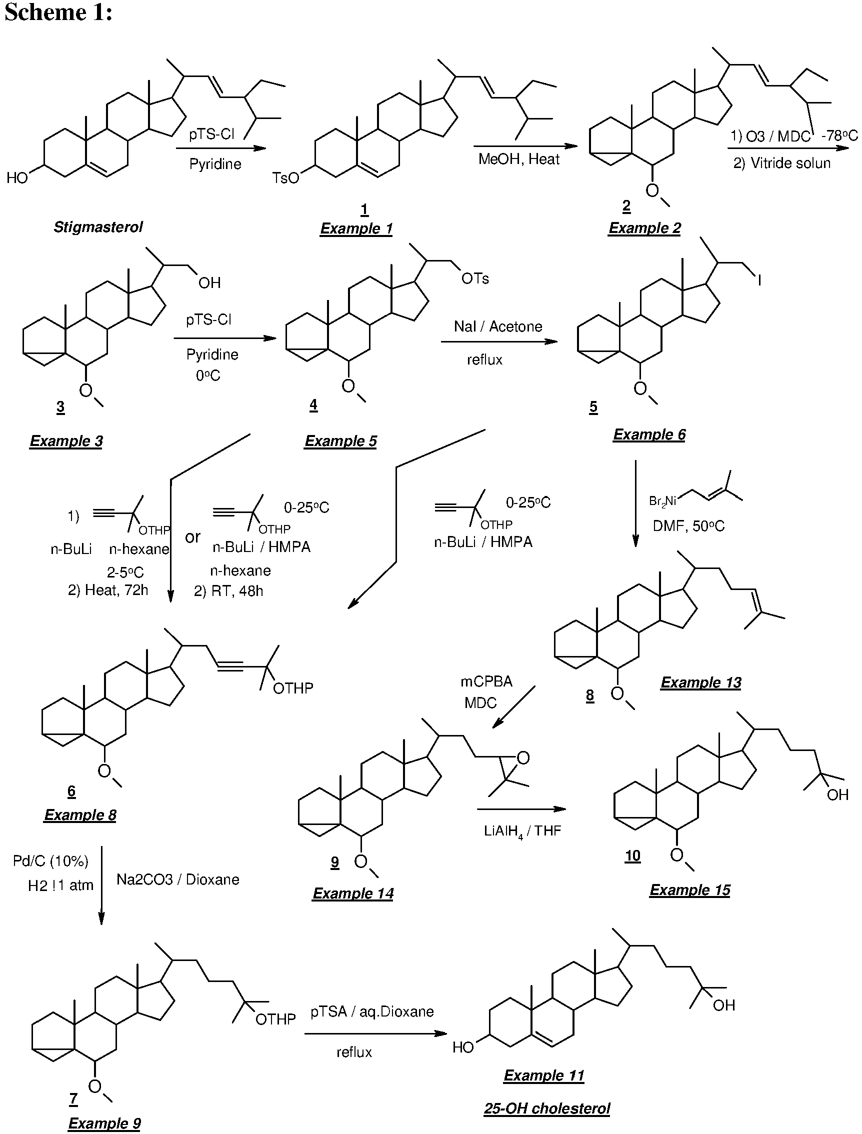

The reported separations of pure stigmasterol from phytosterol are relatively laborious and cumbersome in view of close structural relationship of stigmasterol with other sterols in the mixture viz., beta-sitosterol, campesterol and stigmastenol.

There is literature available for the preparation of 25hydroxycholesterol and esters thereof other than phytosterol.

AU1034095 discloses a process for producing a 25-hydroxycholesterol by hydroxylating cholesterol at the 25-position using ruthenium compound as a catalyst, however, the source of cholesterol is not disclosed.

US3846455A discloses a process for the preparation of 25hydroxycholesterol and esters thereof, which comprises, a) treating fucosterol-24(28)-epoxide or esters thereof with stannic chloride; b) the formation of desmosterol-24(25)-epoxide or 3-esters thereof by treating the desmosterol or esters thereof obtained by the preceding step, with a per organic acid; and, c) the formation of 25-hydroxycholesterol or its 3- ester as the final product by treating the desmosterol- 24(25)-epoxide or 3-ester thereof obtained by the above step (b), with a complex compound of an alkali metal hydride and, if desired, further reacting the resulting 25-hydroxycholesterol with an esterifying agent.

US4183852A discloses synthesis of 25-hydroxycholesterol and 25-hydroxycholecalciferol from animal bile starting materials in which hyodeoxycholic acid or an ester thereof is converted to the 3β-hydroxy-5-cholenic acid alkyl ester, and this is converted to 3β-hydroxy-25-cyano-5-cholene by a series of steps by which the sterol nucleus is stabilized by placing a protecting group at the 3 position and then extending the chain from the carbon at the 24 position to a cyanide group at the 25 position. The compound so formed is subjected to a series of reactions by which it is transformed into 25-hydroxy-7-dehydrocholesterol which may then be irradiated with ultraviolet light to 25-hydroxycholecalciferol. QianZhao et al report synthesis of 25-hydroxycholesterol from desmosterol (Steroids, Volume 85, July 2014, Pages 1-5). The desmosterol was brominated in THF-water (4:1, v/v) using NBS as the brominating agent, followed by the reduction of C-Br with lithium aluminum hydride in THF, to obtain product, 25-hydroxycholesterol with yields and regioselectivity. The reaction is shown in scheme 2 below.

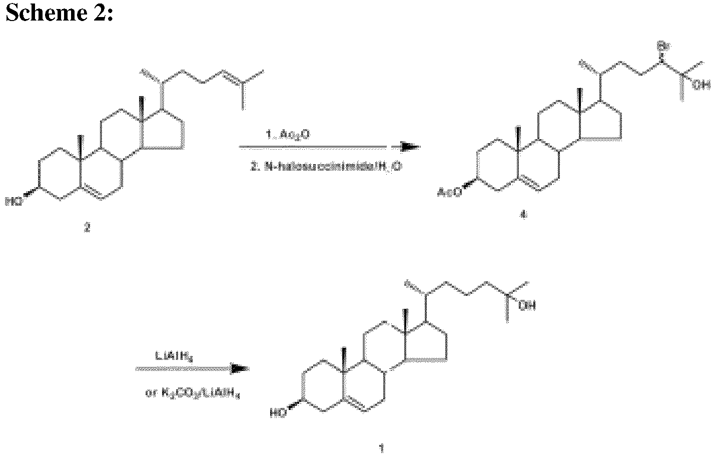

Another method is reported in IN 201717006223, for synthesizing 25-hydroxy cholesterol, characterized in that: adding a 24-dehydrocholesterol derivative and a hydroxyl containing reagent to an organic solvent and react at -40°C~150°C for 1~40 hours in the presence of a catalyst; after completing the reaction, 5 hydrolyzing with alkali, and then separating the reaction solution to obtain 25-hydroxy cholesterol, wherein the structural formulas of the 25-hydroxy cholesterol, the 24-dehydrocholesterol derivative and the hydroxyl containing reagent are shown in formula (I), formula (II) and formula (III) respectively: 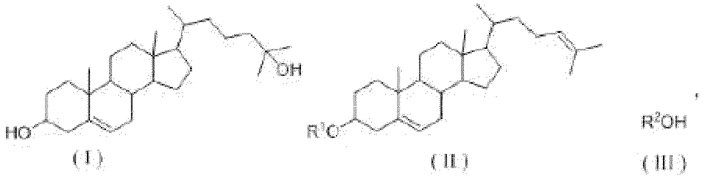 wherein, the R1 in formula (II) is the same as the R2 in formula (III), which is H or C1-C12 acyl group.

Plant sterols and plant stanols, known commonly as phytosterols, are plant-derived compounds that are structurally related to cholesterol and abundant in nature and hence the synthesis of 25-OH cholesterol/Calcifediol from phytosterol will be cost-effective as it is readily available and inexpensive.

J. Org. Chem. Vol 70(21) pp 8513-8521 (2005) discloses a process for the preparation of 25-hydroxy-vitamin D₃, involving reaction of a 20-tosyloxy intermediate with an alkynyl-lithium reagent, followed by 7-bromination and subsequent dehydrobromination and then irradiation and isomerisation. Therefore, the objective of the present invention is to provide a cost-effective and robust process for the preparation of 25-OH cholesterol/Calcifediol from phytosterol.

### Summary of the present invention:

In line with the above objective, the present invention provides a process for preparation of 25-OH cholesterol (7) comprising the steps of:
a) Reacting (20S)-20-hydroxymethyl-6β-methoxy-3α,5-cyclo-5α-pregnane **(3)** with Iodine and imidazole in presence of triphenyl phosphine to obtain (20S)-20-iodo methyl - 6 β- methoxy-30,5-cyclo-5a-pregnane **(5);**
b) Reacting the (20S)-20-iodo methyl - 6 β- methoxy-30,5-cyclo-5a-pregnane **(5)** with ethyl acrylate in presence of a reducing agent (preferably zinc dust and nickel chloride) and a base (preferably pyridine) to obtain Ethyl 1(S)-6β-methoxy-3α,5-cyclo-5α-cholesta-25-oate **(6);** and
c) Reacting the Ethyl 1(S)-6β-methoxy-3α,5-cyclo-5α-cholesta-25-oate (6) with methyl magnesium bromide in a solvent at room temperature under N2 followed by treating with a dehydrating agent in a solvent to obtain 25-OH cholesterol **(7).**

In a process variant, the invention provides process for preparation of 25-OH cholesterol which process comprises;
a) Preparing a solution of magnesium turnings in presence of 1,2-dibromoethane and 4-bromo-2-methyl-2-[(trimethylsilyl)oxy]butane in a solvent at 40 to 60°C under N2;
b) Reacting the contents of step a) with (20S)-6β-methoxy-20-(p-toluene sulfonoxymethyl)- 3α, 5-cyclo-5α-pregnane **(4)** and CuBr•Me2S solution in a solvent to obtain 25-OH cholesterol **(7).**

In another aspect, the invention provides a process for preparation of 25-OH Vitamin D3 (Calcifediol) from 25-OHcholesterol, which process comprises;
a) Preparing 25-OH cholesterol (7) according to the method described above;
b) Reacting 25-OH cholesterol **(7)** with acetic anhydride in presence of a base at 30-50°C to obtain 25-OH cholesterol diacetate **(8);**
c) Brominating the 25-OH cholesterol diacetate with DDH (1,3-dibromo-5,5-dimethyl hydantoin) in presence of an initiator at 50 to 80°C to obtain 25-OH 7-bromo cholesteryl diacetate;
d) Treating the 25-OH 7-bromo cholesteryl diacetate with TBAF in a solvent at 10 to 30°C to obtain 25-OH 7-dehydrocholesteryl diacetate followed by subjecting the 25-OH 7-dehydrocholesteryl diacetate to alkaline hydrolysis at about 30 to 60°C to obtain 25-OH 7-dehydrocholesterol **(9);**
e) Irradiating the 25-Hydroxy-7-dehydrocholesterol **(9)** in THF under high pressure mercury lamp in presence of a sensitizer to obtain 25-hydroxy-pre D3; and
f) Heating the solution containing 25-hydroxy-pre D3 to convert 25-hydroxy-pre D3 to 25-OH vitamin D3 **(Calcifediol).**

### Detailed description:

The invention will now be described in detail in connection with certain preferred and optional embodiments, so that various aspects thereof may be more fully understood and appreciated.

Accordingly the present invention provides process for preparation of 25-OH cholesterol, and finally Calcifediol directly from readily available inexpensive crude phytosterol, as shown in schemes 3 and 4.

Preparation of Phytosteryl tosylate (1); Phytosteryl-i- methyl ether (2); (20S)-20-hydroxymethyl-6β-methoxy-3α,5-cyclo-5α-pregnane (3); (20S)-6β-methoxy-20-(p-toluene sulfonoxy methyl)- 3α,5-cyclo-5α-pregnane (4), starting from crude phytosterol as demonstrated in examples 1 to 4, are prepared as per teachings of US3822254.

In an embodiment of the present invention, the invention provides a process for preparation of 25-OH Vit D3 (Calcifediol), which comprises;
a) Reacting (20S)-20-hydroxymethyl-6β-methoxy-3α,5-cyclo-5α-pregnane **(3)** with Iodine and imidazole in presence of triphenyl phosphine to obtain (20S)-20-iodo methyl - 6 β- methoxy-30,5-cyclo-5a-pregnane **(5);**
b) Reacting the (20S)-20-iodo methyl - 6 β- methoxy-30,5-cyclo-5a-pregnane **(5)** with ethyl acrylate in presence of a reducing agent and a base to obtain Ethyl 1(S)-6β-methoxy-3α,5-cyclo-5α-cholesta-25-oate **(6);**
c) Reacting the Ethyl 1(S)-6β-methoxy-3α,5-cyclo-5α-cholesta-25-oate **(6)** with methyl magnesium bromide in a solvent, at room temperature under N₂ followed by treating with a dehydrating agent in a solvent to obtain 25-OH cholesterol **(7);**
d) Reacting the 25-OH cholesterol **(7)** with acetic anhydride in presence of a base at 30-50°C to obtain 25-OH cholesterol diacetate **(8);**
e) Brominating the 25-OH cholesterol diacetate with brominating agent in presence of an initiator at 50 to 80°C to obtain 25-OH 7-bromo cholesteryl diacetate;
f) Treating the 25-OH 7-bromo cholesteryl diacetate with TBAF in a solvent at 10 to 30°C to obtain 25-OH 7-dehydrocholesteryl diacetate followed by subjecting the 25-OH 7-dehydrocholesteryl diacetate to alkaline hydrolysis at about 30 to 60°C to obtain 25-OH 7-dehydrocholesterol **(9);**
g) Irradiating the 25-Hydroxy-7-dehydrocholesterol **(9)** in a solvent under high pressure mercury lamp in presence of a sensitizer to obtain 25-hydroxy-pre vitamin D3; and
h) Heating the solution containing 25-hydroxy-pre D3 to convert 25-hydroxy-pre D3 to 25-OH vitamin D3 **(Calcifediol).**

The reaction in step b) is carried out at a temperature range of 40 to 70°C.

In a preferred embodiment, the reducing agent in step b) is combination of zinc dust and nickel chloride. However, any other suitable reducing agents such as sodium borohydride, sodium hydride, metal catalysts such as palladium or platinum catalysts etc. can be employed to achieve the desired result.

The heating in step c) is carried out at a temperature range of 50 to 90°C.

In a preferred embodiment, the dehydrating agent in step c) is para toluene sulphonic acid. However, any other suitable dehydrating agents such as sulphuric acid, phosphorous pentoxide, calcium oxide, orthoformic acid, etc. can be employed to achieve the desired result.

The base may be selected from organic or inorganic base. The base is preferably an organic base selected from pyridine, DMAP or combinations thereof. However, any other suitable organic or inorganic bases can be employed to achieve the desired result.

The solvent may be selected from THF, diethylether, dioxane or any other suitable solvents such as acetone, ethylacetate, methylene chloride, ethylene dichloride, acetonitrile, toluene, xylene etc.

In a preferred embodiment, the initiator in step e) is Bis(tert-butylcyclohexyl) peroxydicarbonate and the brominating agent is DDH( 1,3-dibromo-5,5-dimethyl hydantoin). However, any other suitable initiators such as AIBN and brominating agents such as NBS can be employed to achieve the desired result.

In a preferred embodiment, the irradiation sensitizer used in step g) is 5-(3-pyridyl)-2,2'-bithiophene. However, any other suitable irradiation sensitizer such as anthracene, 9-acetyl anthracene can be employed to achieve the desired result.

In a preferred embodiment, the solvent is selected from ethers such as THF, diethylether, dioxane. However, any other suitable solvents can be employed to achieve the desired result.

The process for preparation of 25-OH vitamin **D3(Calcifediol)** is shown below in scheme 3.

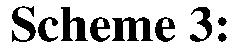

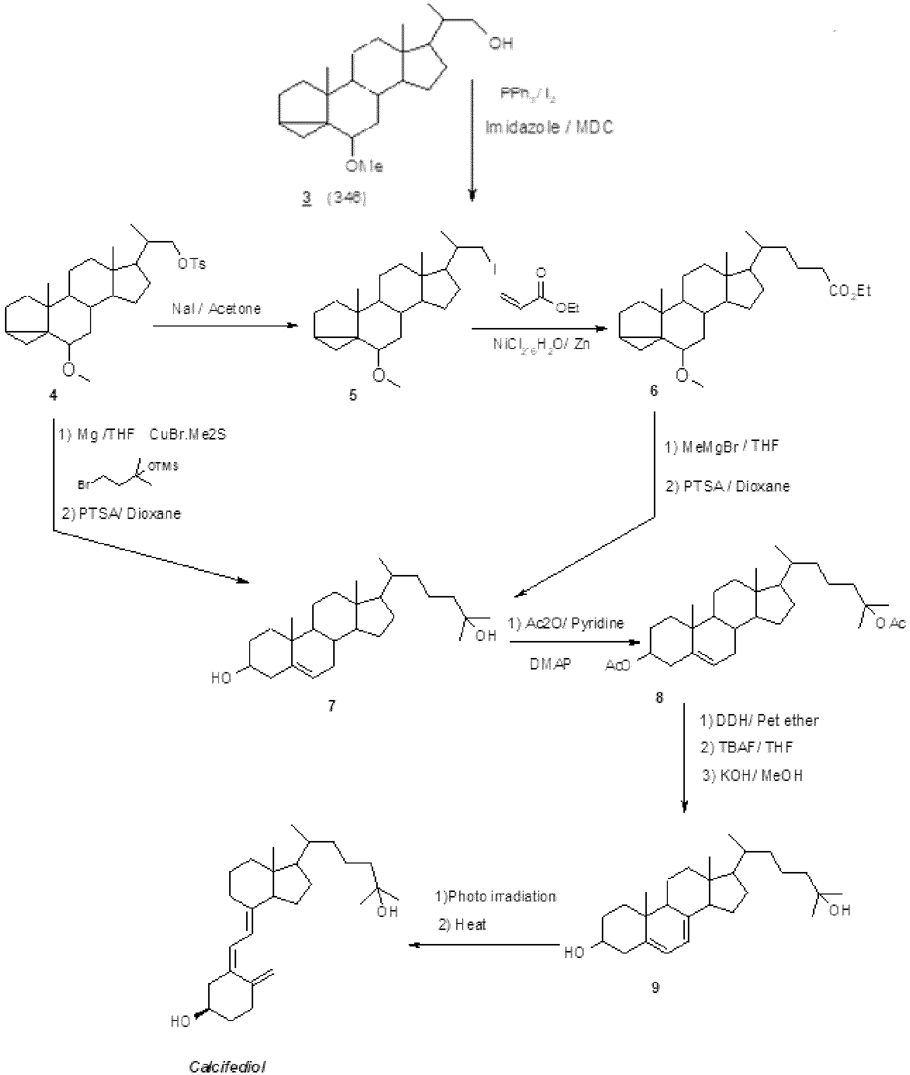

In a claimed embodiment, the invention provides a process for preparation of 25-OH cholesterol (7) comprising the steps of:
a) Reacting (20S)-20-hydroxymethyl-6β-methoxy-3α,5-cyclo-5α-pregnane **(3)** with Iodine and imidazole in presence of triphenyl phosphine to obtain (20S)-20-iodo methyl - 6 β- methoxy-30,5-cyclo-5a-pregnane **(5);**
b) Reacting the (20S)-20-iodo methyl - 6 β- methoxy-30,5-cyclo-5a-pregnane **(5)** with ethyl acrylate in presence of a reducing agent (preferably zinc dust and nickel chloride) and a base (preferably pyridine) to obtain Ethyl 1(S)-6β-methoxy-3α,5-cyclo-5α-cholesta-25-oate **(6)** ; and
c) Reacting the Ethyl 1(S)-6β-methoxy-3α,5-cyclo-5α-cholesta-25-oate (6) with methyl magnesium bromide in a solvent at room temperature under N2 followed by treating with a dehydrating agent in a solvent to obtain 25-OH cholesterol **(7).**

The reaction in step b) is carried out at a temperature range of 40 to 70°C.

The reducing agent in step b) is combination of zinc dust and nickel chloride.

The heating in step c) is carried out at a temperature range of 50 to 90°C.

The base in steps b) and step d) is selected from pyridine, DMAP or combinations thereof.

The process the dehydrating agent in step c) is para toluene sulphonic acid.

In a process variant, the invention discloses a process for preparation of 25-OH cholesterol **(7)** which comprises:
a) Preparing a solution of magnesium turnings in presence of 1,2-dibromoethane and 4-bromo-2-methyl-2-[(trimethylsilyl)oxy]butane in a solvent at 40 to 60°C under N2;
b) Reacting the contents of step a) with (20S)-6β-methoxy-20-(p-toluene sulfonoxymethyl)- 3α, 5-cyclo-5α-pregnane **(4)** and CuBr•Me2S solution in a solvent to obtain 25-OH cholesterol **(7).**

In an embodiment, the solvent is selected from THF, diethylether, dioxane.

However, any other suitable solvents can be employed to achieve the desired result.

The reaction scheme for the synthesis of 25-OH-cholesterol is shown in scheme 4.

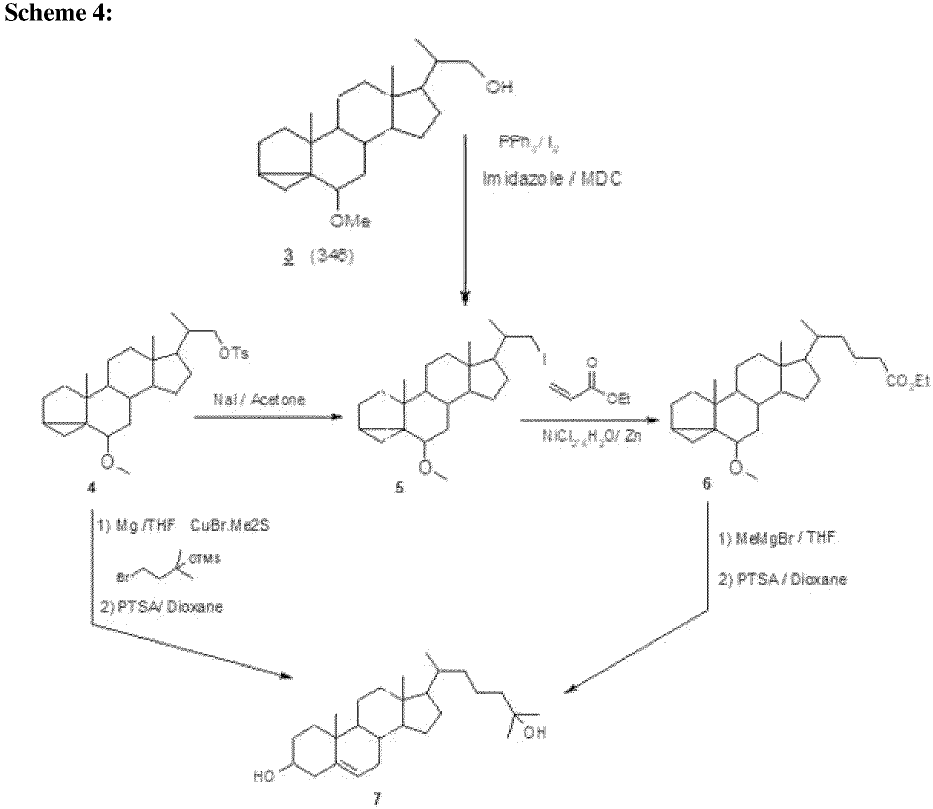

In yet another claimed embodiment, the invention provides a process for preparation of 25-OH Vitamin D3 (Calcifediol) from 25-OHcholesterol (scheme 3), which process comprises;
a) Preparing 25-OH cholesterol (7) as indicated above and in claim 1, and then reacting it with acetic anhydride in presence of a base at 30-50°C to obtain 25-OH cholesterol diacetate (8);
b) Brominating the 25-OH cholesterol diacetate with DDH( 1,3-dibromo-5,5-dimethyl hydantoin) in presence of an initiator at 50 to 80°C to obtain 25-OH 7-bromo cholesteryl diacetate;
c) Treating the 25-OH 7-bromo cholesteryl diacetate with TBAF in a solvent at 10 to 30°C to obtain 25-OH 7-dehydrocholesteryl diacetate followed by subjecting the 25-OH 7-dehydrocholesteryl diacetate to alkaline hydrolysis at about 30 to 60°C to obtain 25-OH 7-dehydrocholesterol **(9);**
d) Irradiating the 25-Hydroxy-7-dehydrocholesterol **(9)** in THF under high pressure mercury lamp in presence of a sensitizer to obtain 25-hydroxy-pre D3; and
e) Heating the solution containing 25-hydroxy-pre D3 to convert 25-hydroxy-pre D3 to 25-OH vitamin D3 **(Calcifediol).**

The base is selected from pyridine, DMAP or combinations thereof and the solvent is selected from THF, diethylether, dioxane.

The irradiation sensitizer used in step d) is selected from 5-(3-pyridyl)-2,2'-bithiophene, anthracene and 9-acetyl anthracene.

The initiator used in the above process is selected from Bis(tert-butylcyclohexyl) peroxydicarbonate or AIBN and the brominating agent is selected from DDH( 1,3-dibromo-5,5-dimethyl hydantoin) and NBS.

In a further embodiment described but not claimed, the invention provides a process for preparation of 25-OH- 7-dehydrocholesterol (9) which process comprises;
a) Reacting 25-OH cholesterol **(7)** with acetic anhydride in presence of base at 30-50°C to obtain 25-OH cholesterol diacetate **(8);**
b) Brominating 25-OH cholesterol diacetate with DDH( 1,3-dibromo-5,5-dimethyl hydantoin) in presence of an initiator at 50 to 80°C to obtain 25-OH 7-bromo cholesteryl diacetate; and
c) Treating the 25-OH 7-bromo cholesteryl diacetate with TBAF in a solvent at 10 to 30°C to obtain 25-OH 7-dehydrocholesteryl diacetate followed by subjecting the 25-OH 7-dehydrocholesteryl diacetate to alkaline hydrolysis at about 30 to 60°C to obtain 25-OH 7-dehydrocholesterol **(9).**

The base may be selected from organic or inorganic base. The base is preferably an organic base selected from pyridine, DMAP or combinations thereof and the solvent is selected from THF, diethylether, dioxane or any other suitable solvents such as acetone, ethylacetate, methylene chloride, ethylene dichloride, acetonitrile, toluene, xylene etc.

The initiator is selected from Bis(tert-butylcyclohexyl) peroxydicarbonate or AIBN and the brominating agent is selected from DDH (1,3-dibromo-5,5-dimethyl hydantoin) or NBS.

In yet another embodiment described but not claimed, the invention encompasses a novel intermediate compound of formula 6, i.e., Ethyl 1(S)-6β-methoxy-3α,5-cyclo-5α-cholesta-25-oate, structurally as shown below:

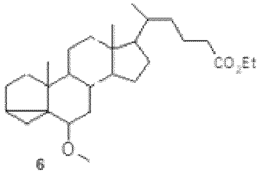

In yet another embodiment described but not claimed, the invention encompasses a novel intermediate compound of formula (8), i.e, 25-OH cholesteryl diacetate, structurally as shown below:

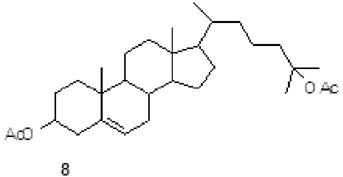

In yet another embodiment described but not claimed, the invention encompasses a novel intermediate compound of formula (9), i.e, 25-OH 7-dehydrocholesterol, structurally as shown below:

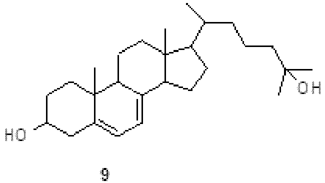

The compound 6, 8 and 9 are further characterised by NMR, IR, Mass spectroscopy.

The invention further encompasses novel intermediate, 25-OH 7-bromo cholesteryl diacetate, however is not isolated due to instability of this compound.

The present invention is exemplified by the following examples which are provided for illustration only and, should not be construed to limit the scope of the invention.

### Examples

### Example 1

### Preparation of Phytosteryl tosylate (1)

To a solution of 500.0 g(1.20mole) of Phytosterol in 5000 ml of dry pyridine was added 500.0 g (2.62 mole) of p-toluene sulfonyl chloride and the mixture was stirred at 25° C for 16 hrs. Pyridine was removed by vacuum distillation and the residue was slowly poured into 10% sodium carbonate solution. The precipitated product was collected by filtration, washed with water followed by methanol and dried in vacuum overnight to yield 600.0 g. of phytosteryl tosylate used for next step without further purification.
Yield: 600 g (88 %)
Appearance: White solid
GC analysis: Stigmasteryl tosylate: 20.37% (RT: 6.10)
   Sitosteryl tosylate: 42.29% (RT:6.80)
   Campesteryl tosylate: 15.60%(RT:5.76)

### Example 2

### Preparation of Phytosteryl-i- methyl ether (2)

A mixture of 600.0 g (1.06 mole) of phytostery tosylate in 5500 ml of methanol and 300 g (3.79 mole) of pyridine was stirred at 55°C for 5 hrs. The cooled solution was concentrated under reduced pressure. The residue was poured into water and extracted with dichloromethane. The dichloromethane solution was dried over anhydrous sodium sulfate and evaporated to dryness to yield 420.0 g. of colorless thick oil used for next step without further purification.
Yield: 420 g (93 %)
Appearance: colorless thick oil
GC analysis: Stigmasteryl-i-methyl ether: 19.49% (RT: 5.47)
   Sitosteryl-i-methyl ether: 48% (RT 6.05)
   Campesteryl-i-methylether: 15.08% (RT: 6.34)

### Example 3

### Preparation of (20S)-20-hydroxymethyl-6β-methoxy-3α,5-cyclo-5α-pregnane (3)

A solution of 420.0 g (0.98 mole) of phytosteryl-i-methyl ether in 4000 ml of methylene chloride and 1300 ml of methanol was cooled to -78⁰ C and treated with ozonized oxygen for 3-4 h. The reaction vessel was flushed with nitrogen and 42 g (1.11 mole) of sodium borohydride was added. The mixture was stirred at -50° C for 1 h and then allowed to warm to 0° C over a 1 h period. Water was added slowly to decompose the excess hydride and the product was extracted with methylene chloride. The methylene chloride solution was washed with brine solution. The methylene chloride solution was then dried over anhydrous sodium sulfate and evaporated to dryness. The 400 g of crude reaction mass was purified by column chromatography using silica gel to get 60.0 g. of (20S)-20-hydroxy methyl-6β-methoxy-3α,5-cyclo-5α-pregnane.
Yield: 60 g (90 %)
Appearance: colorless solid
GC analysis: 93.6% purity (RT 3.35)
Fractions containing sitosterol- i-methyl ether collected separately and concentrated to get thick oil which upon heating in aqueous dioxane in presence of catalytic PTSA at 100°C for 2h, followed by removal of solvent and crystallization in methanol gave 230 g of colourless solid as stigmasterol free form phytosterol.
Yield: 230 g(90%)
Appearance: colorless solid
GC analysis: >95% purity

### Example 4

### Preparation of (20S)-6β-methoxy-20-(p-toluene sulfonoxy methyl)- 3α,5-cyclo-5α-pregnane (4)

To a solution of 60 g (0.172 mole) of (20S)-20 hydroxymethyl-6β-methoxy-3α,5-cyclo-5α-pregnane in 600 ml. of pyridine was added slowly 60 g. (0.314 mole) of p-toluene sulfonyl chloride at 0° C. The mixture was stirred at 0° C for 5 h. Several chips of ice were added and the mixture was stirred for 5 minutes to decompose the excess p-toluene sulfonyl chloride. The mixture was poured into water and the product was extracted with methylene chloride. The methylene chloride solution was washed with water and brine solution. The solution was dried over anhydrous sodium sulfate and evaporated to dryness to yield 78.0 g white solid of (20S)-6β-methoxy-20-(p-toluene sulfonoxy methyl)-3α, 5-cyclo-5α-pregnane used for next step without further purification,
Yield: 78 g (90 %)
Appearance: colorless solid

### Example 5

### Preparation of (20S)-20-iodomethyl-6β-methoxy-3α,5-cyclo-5α-pregnane(5)

A mixture of 78.0 g. (0.156 mole) of (20S)-6β-methoxy-20-(p-toluene sulfonoxy methyl)-3α, 5-cyclo-5α-pregnane, 44.9 g. (0.300 mole) of sodium iodide and 800 ml of dry acetone was heated at reflux for 3 hrs and cooled. The mixture was poured into water and extracted with ethyl acetate. The ethyl acetate extract was dried over anhydrous sodium sulfate to yield 71.0 g of pale yellow solid of (20S)-20-iodo methyl - 63- methoxy-30,5-cyclo-5a-pregnane.
Yield: 71 g (98 %)
Appearance: Pale yellow solid.
M.pt: 102-104°C (rep: 103-104°C)
GC analysis: 95% purity (RT:6.03)
Alternatively (20S)-20-iodomethyl-6β-methoxy-3α,5-cyclo-5α-pregnane (5) was synthesized directly from (20S)-20-hydroxy methyl-6β-methoxy-3α,5-cyclo-5α-pregnane (3) as shown below in example 6.

### Example 6

### Preparation of (20S)-20-iodomethyl-6β-methoxy-3α,5-cyclo-5α-pregnane (5):

Iodine (28.7g, 0.228 mol) was added to a stirred, cooled (0°C) solution of 59g (0.86 mol) of imidazole and 60 g (0.228 mol) of triphenylphosphine in 500 mL of CH2Cl2. The mixture was stirred for 15 min and treated with a solution of 50.0 g (0.144 mol) of (20S)-20-hydroxy methyl-6β-methoxy-3α,5-cyclo-5α-pregnane **(3)** in 250 mL of CH2Cl2 during 20 min, keeping the temperature below 10°C. Stirring was continued at 5°C for 0.5 h and at room temperature for 2.0 h, and the mixture was filtered. The filter cake was washed with 100 mL of CH2Cl2, and the combined filtrate and washing were washed with 400 mL of 2% sodium thiosulfate, 300 mL of 0.1 N HCl, and 300 mL of brine, dried (over Na2SO4), and evaporated to give a pale yellow semisolid. This was stirred with 1.0 L of Et20 and filtered (to remove most triphenylphosphine oxide) and the filtrate was evaporated to get 62.5g of off white solid.
Yield: 62.5 g (94 %)
Appearance: Off white solid
M.Pt: 102-105°C (rep: 103-104°C)
GC analysis: 98.2% purity (RT:6.03)

### Example 7

### Preparation of 25-OHcholesterol (7) from (20S)-6β-methoxy-20-(p-toluene sulfonoxymethyl)- 3α,5-cyclo-5α-pregnane(4)

To stirred magnesium turnings (18.7 g, 0.78 mol) in THF (750 ml) few drops of 1,2-dibromoethane was added under Nitrogen atmosphere followed by few drops of 4-bromo-2-methyl-2-[(trimethylsilyl)oxy]butane and heated the mixture to 50°C for few minutes to initiate reaction then a remaining solution of 4-bromo-2-methyl-2-[(trimethylsilyl)oxy]butane (312 g, 1.30 mol) in THF ( 250mL) was added drop wise under N2. After being stirred at the same temperature 50°C for 30 min, the reaction mixture was cooled at 0 °C and a suspension of CuBr•Me2S (4.0g, 0.01 mol) was added and a solution of (20S)-6β-methoxy-20-(p-toluene sulfonoxy methyl)- 3α,5-cyclo-5α-pregnane **(4)** (78g, 0.156 mol) in THF (250 mL) was added drop wise at 0 °C under N2. After being stirred at room temperature for 2-3 h, the reaction mixture was poured into saturated aqueous NH4Cl at 0 °C and the aqueous layer was extracted twice with EtOAc. The combined organic layer was washed with saturated aqueous NH4Cl, saturated aqueous NaHCO3 and brine, and dried over MgSO4. The obtained mixture was filtered and concentrated in vacuo. The oily residue was dissolved in aqueous dioxane (9:1) 700mL and heated at 80°C in presence of catalytic PTSA until completion of starting material (~3h). Aqueous work up of reaction mixture, followed by extraction with ethyl acetate and removal of solvent yielded 25-OH cholesterol. Crystallization was carried out in methanol to yield 25-OH cholesterol as white crystalline solid (56g).
Yield: 56g (89 %)
M. pt: 176-178°C
[α]²⁵_{D}: -40° (C=1, CHCl3)
Appearance: White crystalline solid
GC analysis: 96.85% (11.04)
HPLC: 98.62% (4.38)
HNMR:
1H-NMR (400 MHz, CDCl3): δ= 0.67 (s, 3 H), 0.94 (s, 3 H), 0.96 (d, 3 H), 1.12 (s, 6 H), 3.55 (m, 1 H), 5.34 (d, 1 H).

The 1H NMR data correspond to those known from the literature.
13C-NMR:
13C NMR (100 MHz, CDCl3): δ140.7, 121.6, 71.7, 71.1, 56.7, 56.1,50.1, 44.4, 42.3, 42.2, 39.7, 37.2, 36.5, 36.4, 35.7, 32.0 31.8, 31.6,29.3, 29.1, 28.2, 24.2, 21.1, 20.7, 19.3, 18.6,11.8;

### Example 8

### Preparation of 25-OHcholesterol (7) from (20S)-20-iodomethyl-6β-methoxy-3α,5-cyclo-5α-pregnane (5):

### 1) Ethyl 1(S)-6β-methoxy-3α,5-cyclo-5α-cholesta-25-oate (6)

To a vigorously stirred mixture of 25.7 g (0.4 mol) of zinc dust and 25.7 mL (36.0 g 0.36 mol) of ethyl acrylate in 60 mL of pyridine was added 18.0 g (0.075 mol) of NiC126H₂0. The mixture was heated to 50°C, whereupon an exotherm ensued, and stirring was continued at 65°C for 30 min. The resulting reddish-brown mixture was cooled to 25°C and treated during 0.5 h with a solution of 50 g (0.109 mol) of (20S)-20-iodo methyl - 63- methoxy-30,5-cyclo-5a-pregnane **(5)** in 100 mL of pyridine at a rate so as to maintain the temperature below 25°C. The mixture was stirred at 25°C for 4 h, poured into 150 mL of EtOAc, and filtered through a pad of Celite. The pad was washed with EtOAc (2 x 100 mL), and the filtrate and washings were washed with 1.0 N HC1 (4 x150 mL), 200 mL of a solution of EDTA (80.0 g EDTA + 80 g NaHC03 in 1.0 L of H2O), and brine (2 x 100 mL), dried (Na2SO4), and evaporated to give 39 g (83%) of crude **(6),** which was used directly in the next step.

An analytical sample was prepared by an additional purification by silicagel column chromatography using EtOAc: n-heptane (2:98) as an eluent. Concentration of collected pure fractions yielded desired product as thick colourless oil.
Appearance: Colourless thick oil
GC purity: 97.6%(RT:8.83)
HNMR:¹H NMR: δ 0.27 (1H, dd) 0.37 (1H, dd), 0.83 (1H,dddd), 0.87 (6H,s), 1.03 (3H, s), 1.15 (3H, t), 2.23 (1H,t), 2.23 (1H,t), 3.04 (3H, s), 4.00-4.12 (3H, 4.10 (q), 4.04 (1H dd)).
¹³CNMR:
   13C NMR (100 MHz, CDCl3): δ173.9, 82.4, 60.1, 56.5, 56.4, 55.9, 47.9, 43.3, 42.7, 40.2, 35.4, 35.3, 35.2, 35.0,34.7,33.3, 30.4, 28.2, 24.9, 24.1, 22.7, 21.5, 21.4, 19.2,18.5,14.2, 13.0, 12.2;
   Mass spectrum (m/e): 431(M+1), 429(M⁻1)

### 2) 25-OH cholesterol (7)

To a stirred, cooled (ice bath) solution of 39g (0.090mol) of ester **(6)** in 200mL of dry THF under nitrogen was added 52 mL (0.226 mol) of methyl magnesium bromide (3.0 M in ether) during 30 min. The mixture was stirred at ice bath temperature for 15 min and at room temperature for 2-3 h, cooled to 0°C, and carefully quenched with saturated NH4Cl. It was extracted with 2L of EtOAc, washed with brine (3 x 250 mL), dried (Na2SO4), and evaporated in vacuo to give 39.0 g of crude product, stirred with methanol, filtered, dried to get 38 g (82%) of desired product as a color less solid which was dissolved in aqueous dioxane (9:1) 500mL and heated at 80°C in presence of catalytic PTSA until completion of starting material (~3h). Aqueous work up of reaction mixture, followed by extraction with ethyl acetate and removal of solvent yielded 25-OH cholesterol. Crystallization was carried out in methanol gave 30g of 25-OH cholesterol as white crystalline solid.
Yield: 30g(82%)
M.pt: 175-177°C
Appearance: White crystalline solid
GC analysis: >95%(11.04)
HPLC: ~98% (4.38)
HNMR:
1H-NMR (400 MHz, CDCl3): δ= 0.68 (s, 3 H), 0.94 (s, 3 H), 0.97 (d, 3 H), 1.14 (s, 6 H), 3.52 (m, 1 H), 5.4 (d, 1 H).

The 1H NMR data correspond to those known from the literature.

### Example 9

### 25-OH cholesteryl diacetate (8):

25-OH cholesterol(56g, 0.14mole) was dissolved in pyridine (400mL) followed by addition of acetic anhydride (31.36g, 0.30mole) and DMAP(20.0g,0.163 mole) at room temperature and stirred at 40°C for 6h until completion of starting material. Reaction mixture was poured in to cold water and extracted with ethyl acetate (200mL X4), washed the ethyl acetate layer with 1N HCl solution (50mLX4) followed by 5% NaHCO3 solution(200mL), dried the solvent over anhydrous Na2SO4 and removed the solvent to get sticky solid which upon purification by column chromatography gave pure 25-OH cholesterol diacetate as white solid(50.0g).
Yield: 50g (75 %)
M.pt: 120-124°C
Appearance: White solid
HPLC: ~90% (RT:6.12)
GC analysis: 92.5% (RT: 11.3)
1HNMR:
   1H-NMR (400 MHz, CDCl3): δ= 0.94 (s, 3 H), 0.97 (d, 3 H), 1.42 (s, 6 H),2.05-2.07(s,6H),2.32 (1H, dd), 2.31(1H, dd), 4.64 (m, 1 H), 5.38 (dd, 1 H).
   ¹³CNMR:
      13C NMR (100 MHz, CDC13): δ170.5, 170.5, 139.6,122.6, 82.5, 73.9, 56.6, 56.1, 50.0, 42.3, 41.1, 39.7, 38.1, 36.9, 36.5, 36.1,36.1, 35.6, 31.8, 31.8, 28.2, 27.7, 26.0, 24.2, 22.4, 21.4, 21.0, 20.4, 19.2, 18.5 ,11.8;
      Mass spectrum (m/e): 487(M+1)

### Example 10

### Preparation of 25-OH 7-dehydrocholesterol (9):

### 1) 25-OH 7-bromo cholesteryl diacetate

To a stirred solution of 25-OH cholesterol diacetate (50g 0.10mole) in 500mL pet ether was added DDH (1,3-dibromo-5,5-dimethyl hydantoin (16.4g, 0.057mole) followed by catalytic perkadox (Bis(tert-butylcyclohexyl) peroxydicarbonate) as initiator. Reaction mixture was heated to reflux at 65°C, until completion of starting material, monitored by TLC and HPLC. Reaction was quenched by adding water and extracted with pet ether, washed the pet ether layer with water, dried the pet ether layer over anhydrous Na2SO4 and removed the solvent to get crude 7- bromo 25-OH cholesterol diacetate as a pale yellow thick oil (~ 50g), used as such for next reaction without further purification.
HPLC: 60% (RT 5.95)

### 2) 25-OH 7-dehydrocholesteryl diacetate

To a stirred cooled solution of 7-Bromo 25-OH cholesterol diacetate (50g, 0.176mole) in dry THF (200mL) was added anhydrous TBAF (100g, 0.317 mole) dissolved in dry THF (400mL). Stirred the reaction mixture at 20oC for 2hrs. Reaction was monitored by HPLC. Reaction was quenched by adding water and extracted with ethyl acetate (3X200mL), washed the ethyl acetate layer with brine solution ( 200mL) and dried over anhydrous Na2SO4 and removed the solvent to get crude as colourless solid. The solid was stirred with methanol, filtered the solid, dried and used for saponification step.
Yield: 30 g (60 %)
M.pt: 129-131°C
Appearance: White solid
HPLC: >95%(RT:7.18)
GC analysis: 98.8% (RT: 12.04)
1H-NMR (400 MHz, CDCl3): δ=0.94 (s, 3 H), 0.95 (d, 3 H), 1.42 (s, 6 H), 2.05-2.07(s,6H), 2.53(1H, dd), 2.39(1H, dd), 4.74 (m, 1 H), 5.39 (dd, 1 H), 5.58(dd,1H)
¹³CNMR:
   13C NMR (100 MHz, CDCl3): δ170.5, 170.5, 141.5, 138.5, 120.2, 116.3, 82.5, 72.8, 55.9, 54.4, 46.0, 42.9, 41.1, 39.1, 37.9, 37.1, 36.6, 36.1,36.0, 28.1, 26.1, 26.0, 22.9, 22.5, 21.4, 21.0, 20.5, 18.7,16.2,11.8;
   Mass spectrum (m/e): 485(M+1)

### 3) 25-OH 7-dehydrocholesterol

25-OH 7-dehydrocholesteryl diacetate (30 g, 0.62mole) was suspended in methanol (300mL) and KOH (20g, 0.36mole) added and heated the reaction mixture at 45°C for 2h. Reaction was monitored by TLC. Solvent was removed by distillation and water was added to residual mass and extracted with dichloromethane. Dichloromethane layer was washed with brine and dried over anhydrous Na2SO4, filtered and solvent was removed to get crude product. The crude product was crystallized in methanol/ acetone to get pure 25-OH-7-dehydrocholesterol as white solid (25g).
Yield: 25 g (80 %)
M.pt: 187-191°C
Appearance: White solid
HPLC: ~98%(RT:9.79)
HNMR:
1H-NMR (400 MHz, CDCl3): δ= 0.62 (s, 3 H), 0.94 (s, 3 H), 0.95 (d, 3 H), 1.12 (s, 6 H), 3.66 (m, 1 H), 5.39 (dd, 1 H), 5.58(dd,1H)
¹³CNMR:
   13C NMR (100 MHz, CDCl3): δ141.3,139.8, 119.6, 116.3,71.1, 70.4, 55.8, 54.5,46.2, 44.4, 42.9, 40.8, 39.1, 38.4, 37.0, 36.4, 36.1, 32.0 29.4,29.2, 28.1, 23.0, 21.1, 20.8, 18.8, 16.3,11.8;

## Claims

1. A process for preparation of 25-OH cholesterol (7) comprising the steps of:
a) Reacting (20S)-20-hydroxymethyl-6β-methoxy-3α,5-cyclo-5α-pregnane (3) with Iodine and imidazole in presence of triphenyl phosphine to obtain (20S)-20-iodo methyl - 6 β- methoxy-30,5-cyclo-5a-pregnane (5);
b) Reacting the (20S)-20-iodo methyl - 6 β- methoxy-30,5-cyclo-5a-pregnane (5) with ethyl acrylate in presence of a reducing agent and a base to obtain Ethyl 1(S)-6β-methoxy-3α,5-cyclo-5α-cholesta-25-oate (6); and
c) Reacting the Ethyl 1(S)-6β-methoxy-3α,5-cyclo-5α-cholesta-25-oate (6) with methyl magnesium bromide in a solvent at room temperature under N2 followed by treating with a dehydrating agent in a solvent to obtain 25-OH cholesterol (7).

2. The process as claimed in claim 1, wherein the reaction in step b) is carried out at a temperature range of 40 to 70°C, or
wherein, the reducing agent in step b) is combination of zinc dust and nickel chloride, or
wherein the heating in step c) is carried out at a temperature range of 50 to 90°C, or
wherein the base in steps b) is selected from pyridine, DMAP or combinations thereof, or
wherein the dehydrating agent in step c) is para toluene sulphonic acid.

3. The process as claimed in claim 1, wherein the 25-OH cholesterol (7) is prepared by a process comprising the steps of:
a) Preparing a solution of magnesium turnings in presence of 1,2-dibromoethane and 4-bromo-2-methyl-2-[(trimethylsilyl)oxy]butane in a solvent at 40 to 60°C under N2;
b) Reacting the contents of step a) with (20S)-6β-methoxy-20-(p-toluene sulfonoxymethyl)- 3α, 5-cyclo-5α-pregnane (4) and CuBr•Me2S solution in a solvent to obtain 25-OH cholesterol (7).

4. The process as claimed in claim 1 or claim 3, wherein the solvent is selected from THF, diethylether, dioxane.

5. A process for preparation of 25-OH Vit D3(Calcifediol) which comprises;
a) preparing 25-OH cholesterol (7) according to the method of any one of claims 1 or 2;
b) Reacting the 25-OH cholesterol (7) with acetic anhydride in presence of a base at 30-50°C to obtain 25-OH cholesterol diacetate (8);
c) Brominating the 25-OH cholesterol diacetate with brominating agent in presence of an initiator at 50 to 80°C to obtain 25-OH 7-bromo cholesteryl diacetate;
d) Treating the 25-OH 7-bromo cholesteryl diacetate with TBAF in a solvent at 10 to 30°C to obtain 25-OH 7-dehydrocholesteryl diacetate followed by subjecting the 25-OH 7-dehydrocholesteryl diacetate to alkaline hydrolysis at about 30 to 60°C to obtain 25-OH 7-dehydrocholesterol (9);
e) Irradiating the 25-Hydroxy-7-dehydrocholesterol (9) in a solvent under high pressure mercury lamp in presence of a sensitizer to obtain 25-hydroxy-pre vitamin D3; and
f) Heating the solution containing 25-hydroxy-pre D3 to convert 25-hydroxy-pre D3 to 25-OH vitamin D3 (Calcifediol).

6. The process as claimed in claim 5,
wherein the base in step d) is selected from pyridine, DMAP or combinations thereof.

7. The process as claimed in claim 5,
wherein the initiator is selected from Bis(tert-butylcyclohexyl) peroxydicarbonate or AIBN and the brominating agent is selected from DDH (1,3-dibromo-5,5-dimethyl hydantoin) or NBS, or
wherein the irradiation sensitizer used in step e) is selected from 5-(3-pyridyl)-2,2'-bithiophene; anthracene or 9-acetyl anthracene, or
wherein the solvent is selected from THF, diethylether, dioxane.

## Patentansprüche

1. Verfahren zur Herstellung von 25-OH-Cholesterol (7), umfassend folgende Schritte:
a) Reagierenlassen von (20S)-20-Hydroxymethyl-6β-methoxy-3α,5-cyclo-5α-pregnan (3) mit lod und Imidazol in Gegenwart von Triphenylphosphin zum Erhalten von (20S)-20-Iodomethyl-6β-methoxy-30,5-cyclo-5a-pregnan (5);
b) Reagierenlassen von (20S)-20-Iodomethyl-6β-methoxy-30,5-cyclo-5a-pregnan (5) mit Ethylacrylat in Gegenwart eines Reduktionsmittels und einer Base zum Erhalten von Ethyl-1 (S)-6β-methoxy-3α,5-cyclo-5α-cholesta-25-oat (6); und
c) Reagierenlassen von Ethyl-1 (S)-6β-methoxy-3α,5-cyclo-5α-cholesta-25-oat (6) mit Methylmagnesiumbromid in einem Lösungsmittel bei Zimmertemperatur unter N2 und anschließendes Behandeln mit einem Dehydrierungsmittel in einem Lösungsmittel zum Erhalten von 25-OH-Cholesterol (7).

2. Verfahren nach Anspruch 1, wobei die Reaktion in Schritt b) in einem Temperaturbereich von 40 bis 70 °C durchgeführt wird, oder
wobei das Reduktionsmittel in Schritt b) eine Kombination aus Zinkstaub und Nickelchlorid ist oder
wobei das Erwärmen in Schritt c) in einem Temperaturbereich von 50 bis 90 °C durchgeführt wird, oder
wobei die Base in Schritt b) aus Pyridin, DMAP oder Kombinationen davon ausgewählt wird oder
wobei das Dehydrierungsmittel in Schritt c) para-Toluolsulphonsäure ist.

3. Verfahren nach Anspruch 1, wobei 25-OH-Cholesterol (7) mit einem Verfahren hergestellt wird, umfassend folgende Schritte:
a) Herstellen einer Lösung aus Magnesiumspänen in Gegenwart von 1,2-Dibromethan und 4-Brom-2-methyl-2-[(trimethylsilyl)oxy]butan in einem Lösungsmittel bei 40 bis 60 °C unter N2;
b) Reagierenlassen des Inhalts von Schritt a) mit (20S)-6β-Methoxy-20-(p-toluolsulfonoxymethyl)-3α,5-cyclo-5α-pregnan (4) und CuBr·Me2S-Lösung in einem Lösungsmittel zum Erhalten von 25-OH-Cholesterol (7).

4. Verfahren nach Anspruch 1 oder Anspruch 3, wobei das Lösungsmittel aus THF, Diethylether, Dioxan ausgewählt ist.

5. Verfahren zur Herstellung von 25-OH-Vit. D3 (Calcifediol), umfassend:
a) Herstellen von 25-OH-Cholesterol (7) gemäß dem Verfahren nach einem der Ansprüche 1 oder 2;
b) Reagierenlassen von 25-OH-Cholesterol (7) mit Essigsäureanhydrid in Gegenwart einer Base bei 30-50 °C zum Erhalten von 25-OH-Cholesteroldiacetat (8);
c) Bromieren von 25-OH-Cholesteroldiacetat mit einem Bromierungsmittel in Gegenwart eines Initiators bei 50 bis 80 °C zum Erhalten von 25-OH-7-Bromcholesteryldiacetat;
d) Behandeln von 25-OH-7-Bromcholesteryldiacetat mit TBAF in einem Lösungsmittel bei 10 bis 30 °C zum Erhalten von 25-OH-7-Dehydrocholesteryldiacetat und anschließende alkalische Hydrolyse von 25-OH-7-Dehydrocholesteryldiacetat bei ungefähr 30 bis 60 °C zum Erhalten von 25-OH-7-Dehydrocholesterol (9);
e) Bestrahlen von 25-Hydroxy-7-dehydrocholesterol (9) in einem Lösungsmittel unter einer Hochdruckquecksilberlampe in Gegenwart eines Sensibilisators zum Erhalten von 25-Hydroxy-Prävitamin D3; und
f) Erwärmen der Lösung, die 25-Hydroxy-prä-D3 enthält, zum Umsetzen von 25-Hydroxy-prä-D3 zu 25-OH-Vitamin D3 (Calcifediol).

6. Verfahren nach Anspruch 5, wobei die Base in Schritt d) ausgewählt ist aus Pyridin, DMAP oder Kombinationen davon.

7. Verfahren nach Anspruch 5,
wobei der Initiator aus Bis(tert-butylcyclohexyl)peroxydicarbonat oder AIBN ausgewählt ist und das Bromierungsmittel aus DDH (1,3-Dibrom-5,5-dimethylhydantoin) oder NBS ausgewählt ist, oder
wobei der Bestrahlungssensibilisator, der in Schritt e) verwendet wird, aus 5-(3-Pyridyl)-2,2'-bithiophen; Anthracen oder 9-Acetylanthracen ausgewählt ist, oder
wobei das Lösungsmittel aus THF, Diethylether, Dioxan ausgewählt ist.

## Revendications

1. Procédé de préparation de 25-OH-cholestérol (7) comprenant les étapes consistant à :
a) faire réagir du (20S)-20-hydroxyméthyl-6β-méthoxy-3α,5-cyclo-5α-prégnane (3) avec de l'iode et de l'imidazole en présence de triphénylphosphine pour obtenir du (20S)-20-iodométhyl-6β-méthoxy-30,5-cyclo-5a-prégnane (5) ;
b) faire réagir le (20S)-20-iodométhyl-6β-méthoxy-30,5-cyclo-5a-prégnane (5) avec de l'éthyl-acrylate en présence d'un agent réducteur et d'une base afin d'obtenir de l'éthyl-1(S)-6β-methoxy-3α,5-cyclo-5α-cholesta-25-oate (6) ; et
c) faire réagir l'éthyl-1(S)-6β-methoxy-3α,5-cyclo-5α-cholesta-25-oate (6) avec du bromure de méthylmagnésium dans un solvant à la température ambiante sous N2 suivi d'un traitement avec un agent déshydratant dans un solvant afin d'obtenir du 25-OH-cholestérol (7).

2. Procédé selon la revendication 1, dans lequel la réaction dans l'étape b) est réalisée à une plage de température comprise entre 40 et 70 °C, ou
dans lequel l'agent réducteur dans l'étape b) est une combinaison de poussière de zinc et de chlorure de nickel, ou
dans lequel le chauffage dans l'étape c) est réalisé à une plage de température comprise entre 50 et 90 °C, ou
dans lequel la base dans l'étape b) est sélectionnée parmi du pyridine, du DMAP ou des combinaisons de ceux-ci, ou
dans lequel l'agent déshydratant dans l'étape c) est de l'acide para-toluène-sulfonique.

3. Procédé selon la revendication 1, dans lequel le 25-OH-cholestérol (7) est préparé par un procédé comprenant les étapes consistant à :
a) préparer une solution de copeaux de magnésium en présence de 1,2-dibromoéthane et de 4-bromo-2-méthyl-2-[(triméthylsilyl)oxy]butane dans un solvant à 40 à 60 °C sous N2 ;
b) faire réagir le contenu de l'étape a) avec le (20S)-6β-méthoxy-20-(p-toluène-sulfonoxyméthyl)-3α,5-cyclo-5α-prégnane (4) et la solution de CuBr•Me2S dans un solvant afin d'obtenir du 25-OH cholestérol (7).

4. Procédé selon la revendication 1 ou la revendication 3, dans lequel le solvant est sélectionné parmi du THF, du diéthyléther, du dioxane.

5. Procédé de préparation de 25-OH Vit D3(Calcifédiol) qui comprend ;
a) la préparation de 25-OH cholestérol (7) selon le procédé selon l'une quelconque des revendications 1 ou 2 ;
b) la réaction du 25-OH cholestérol (7) avec de l'anhydride acétique en présence d'une base à 30-50 °C afin d'obtenir du diacétate de 25-OH cholestérol (8) ;
c) la bromation du 25-OH cholestérol diacétate avec un agent bromant en présence d'un initiateur à 50 à 80 °C afin d'obtenir du diacétate de 25-OH 7-bromo-cholestéryle ;
d) le traitement du diacétate de 25-OH-7-bromo-cholestéryle avec du TBAF dans un solvant à 10 à 30 °C afin d'obtenir du diacétate de 25-OH-7-déhydrocholestéryle suivi de la soumission du diacétate de 25-OH-7-déhydrocholestéryle à une hydrolyse alcaline à environ 30 à 60 °C afin d'obtenir du 25-OH-7-déhydrocholestérol (9) ;
e) l'irradiation du 25-Hydroxy-7-déhydrocholestérol (9) dans un solvant sous une lampe de mercure à haute pression en présence d'un sensibilisant afin d'obtenir de la 25-hydroxy-pré-vitamine D3 ; et
f) le chauffage de la solution contenant de la 25-hydroxy-pré-D3 pour convertir la 25-hydroxy-pré-D3 en 25-OH vitamine D3 (Calcifédiol).

6. Procédé selon la revendication 5, dans lequel la base dans l'étape d) est sélectionnée parmi du pyridine, du DMAP ou de combinaisons de ceux-ci.

7. Procédé selon la revendication 5,
dans lequel l'initiateur est sélectionné parmi du Bis(tert-butylcyclohexyl) peroxydicarbonate ou de l'AIBN et l'agent bromant est sélectionné parmi du DDH-(1,3-dibromo-5,5-diméthyl-hydantoïne) ou du NBS, ou
dans lequel le sensibilisant aux radiations utilisé dans l'étape e) est sélectionné parmi du 5-(3-pyridyl)-2,2'-bithiophène ; de l'anthracène ou du 9-acétyl-anthracène, ou
dans lequel le solvant est sélectionné parmi du THF, du diéthyléther, du dioxane.
